# EUROPÄISCHE PATENTSCHRIFT

(11) **EP 1 979 361 B1**
(45) Veröffentlichungstag und Bekanntmachung des Hinweises auf die Patenterteilung: **24.11.2010**
(21) Anmeldenummer: 07702589.8
(22) Anmeldetag: 05.01.2007
(51) Int. Cl.: C07F 5/02, C07F 9/52, C07C 381/12, C07C 311/48, B01J 31/02

(54) **OXONIUM- UND SULFONIUMSALZE**
OXONIUM AND SULFONIUM SALTS
SELS D'OXONIUM ET DE SULFONIUM

(30) Priorität: 04.02.2006 DE 102006005103
(43) Veröffentlichungstag der Anmeldung: 15.10.2008
(60) Teilanmeldung: 10008348.4
(73) Patentinhaber: Merck Patent GmbH, 64293 Darmstadt (DE)
(72) Erfinder: IGNATYEV, Nikolai (Mykola), 47058 Duisburg (DE); BISSKY, German, 42109 Wuppertal (DE); WILLNER, Helge, 45481 Muelheim/Ruhr (DE)
(86) Internationale Anmeldenummer: PCT/EP2007/000051
(87) Internationale Veröffentlichungsnummer: WO 2007/087949

(56) Entgegenhaltungen:
- EP-A- 1 679 307
- WO-A-2004/002955
- WO-A-2005/116038
- FR-A1- 2 727 416
- GB-A- 2 387 127
- JP-A- 2004 123 631
- JP-A- 2005 275 153
- JP-A- 2006 258 925
- US-A- 2 946 770
- US-A1- 2003 060 359
- US-A1- 2003 148 211
- MATSUMOTO ET AL: "Preparation of room temperature ionic liquids based on aliphatic onium cations and asymmetric amide anions and their electrochemical properties as a lithium battery electrolyte" JOURNAL OF POWER SOURCES, ELSEVIER, AMSTERDAM, NL, Bd. 146, Nr. 1-2, 26. August 2005 (2005-08-26), Seiten 45-50, XP005076585 ISSN: 0378-7753
- LEE D ET AL: "Perfluorosulfonyl imides and methides - Investigating the lithographic potential of novel superacid PAGs" PROCEEDINGS OF THE SPIE, SPIE, BELLINGHAM, VA, US, Bd. 4690, Nr. 1, März 2002 (2002-03), Seiten 169-177, XP002309548 ISSN: 0277-786X
- OKOTURO ET AL: "Temperature dependence of viscosity for room temperature ionic liquids" JOURNAL OF ELECTROANALYTICAL CHEMISTRY AND INTERFACIAL ELECTROCHEMISTRY, ELSEVIER, AMSTERDAM, NL, Bd. 568, 2004, Seiten 167-181, XP002334479 ISSN: 0022-0728
- MATSUMOTO, HAJIME ET AL: "Room temperature molten salts based on trialkylsulfonium cations and bis(trifluoromethylsulfonyl)imide" CHEMISTRY LETTERS , (12), 1430-1431 CODEN: CMLTAG; ISSN: 0366-7022, 2000, XP009088266

## Beschreibung

Die vorliegende Erfindung betrifft ein Verfahren gemäß Anspruch 1.

Ionische Flüssigkeiten stellen durch Ihre Eigenschaften in der modernen Forschung eine wirksame Alternative zu traditionellen flüchtigen organischen Solventien für die organische Synthese dar. Die Verwendung von ionischen Flüssigkeiten als neues Reaktionsmedium könnte weiterhin eine praktische Lösung sowohl für die Lösungsmittel-Emission als auch für Probleme in der Wiederaufbereitung von Katalysatoren sein.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf. Der Schmelzpunkt kann jedoch auch höher liegen, ohne die Anwendbarkeit der Salze auf allen Anwendungsgebieten zu beschränken. Beispiele für organische Kationen sind unter anderem Tetraalkylammonium, Tetraalkylphosphonium, N-Alkylpyridinium, 1,3-Dialkylimidazolium oder Trialkylsulfonium. Unter einer Vielzahl von geeigneten Anionen sind beispielsweise BF₄⁻, PF₆⁻, SbF₆⁻, NO₃⁻, CF₃SO₃⁻, (CF₃SO₂)₂N⁻, ArylSO₃⁻, CF₃CO₂⁻, CH₃CO₂⁻ oder Al₂Cl₇⁻ zu nennen.

Die Eigenschaften der ionischen Flüssigkeiten, beispielsweise der Schmelzpunkt, die thermische und elektrochemische Stabilität oder Viskosität, werden durch die Auswahl der Kationen und Anionen bestimmt. Übersichtsartikel zu ionischen Flüssigkeiten sind beispielsweise R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton "Room temperature ionic liquids. Solvents for synthesis and catalysis", Chem. Rev., 92 (1999), 2071-2083 oder R. Hagiwara, Ya. Ito "Room temperature ionic liquids of alkylimidazolium cations and fluoroanions", J. Fluorine Chem., 105 (2000), 221-227).

Eine große Anzahl von Onium-Salzen mit Perfluoralkylfluorphosphat-, Bis(perfluoralkylsulfonyl)imid-, Perfluoralkylborat- oder Tetracyanoborat-Anionen sind ionische Flüssigkeiten.

Üblicherweise werden entsprechende Onium-Salze mit den oben genannten Anionen durch Anionenaustausch z.B. aus einem Onium-Halogenid in wässrigen Medien erzeugt. Dieser Verfahrensweg erweist sich aber als wenig praktikabel, wenn das Onium-Halogenid gegenüber Wasser nicht stabil ist. Dies gilt beispielsweise für eine Reihe von Oxonium- oder Sulfonium-Kationen.

Es besteht daher ein Bedarf an einem Zugang zu Onium-Salzen mit Perfluoralkylfluorphosphat-, Anionen-, der die oben genannten Nachteile nicht aufweist. Die Bereitstellung eines alternativen Syntheseverfahrens ist demgemäß die Aufgabe der vorliegenden Erfindung.

Ein erster Gegenstand der vorliegenden Erfindung ist ein Verfahren zur Herstellung von Sulfoniumsalzen mit [(R^{o})₃S]⁺-Kationen, wobei R^{o} geradkettige oder verzweigte Alkylgruppen mit 1-8 C-Atomen oder unsubstituiertes oder mit R^{o}, OR^{o}, N(R^{o})_{2,} CN oder Halogen substituiertes Phenyl bedeutet, und [PFₓ(C_{y}F_{2y+1-z}H_{z})₆₋ₓ]⁻-Anionen mit 2≤x≤5, 1≤y≤8 und 0≤z≤2y+1, gemaß Anspruch 1.

R^{o} des [(R^{o})₃S]⁺-Kations ist vorzugsweise geradkettiges Alkyl mit 1-8 C-Atomen, bevorzugt mit 1-4 C-Atomen, insbesondere Methyl oder Ethyl, ganz besonders bevorzugt Ethyl.

Gegenstand der vorliegenden Erfindung ist ein Verfahren zur Herstellung von Sulfoniumsalzen gemäß Anspruch 1 mit [PFₓ(C_{y}F_{2y+1-z}H_{z})₆₋ₓ]⁻-Anionen , wobei ein -[(R^{o})₃S]⁺-Tetrafluoroborat oder -Hexafluorophosphat mit einem Phosphoran der allgemeinen Formel PFₐ(C_{y}F_{2y+1-z}H_{z})₅₋ₐ umgesetzt wird, wobei 1≤a≤4, und R^{o}, y und z die in Anspruch 1 angegebene Bedeutung haben.

Die in dem erfindungsgemäßen Verfahren eingesetzten Phosphorane sind beispielsweise über elektrochemische Fluorierung von Alkylphosphoranen oder Alkylphosphanen zugänglich. Verfahren dieser Art sind dem Fachmann beispielsweise aus EP 1 037 896 bekannt.

In einer alternativen Variante können die Sulfoniumsalze durch Umsetzung eines [(R^{o})₃S]⁺-Tetrafluoroborats oder -Hexafluorophosphats mit H⁺[PFₓ(C_{y}F_{2y+1-z}H_{z})₆₋ₓ]⁻, oder mit einem Alkali- oder Erdalkalimetallsalz von H⁺[PFₓ(C_{y}F_{2y+1-z}H_{z})₆₋ₓ]⁻, wobei R^{o}, x, y, z, die vorab angegebene Bedeutung haben, erhalten werden.

Säuren des Typs H⁺[PFₓ(C_{y}F_{2y+1-z}H_{z})₆₋ₓ]⁻ können nach Syntheseverfahren hergestellt werden, wie sie aus der Literatur, z.B. aus EP 1 399 453, bekannt sind.

Als Sulfonium-Tetrafluoroborat mit der Formel [(R^{o})₃S]⁺ [BF₄]⁻ wird bevorzugt ein Sulfonium-Tetrafluoroborat mit geradkettigen oder verzweigten Alkylgruppen mit 1-8 C-Atomen, bevorzugt mit 1-4 C-Atomen eingesetzt, die jeweils unabhängig voneinander sind. Bevorzugt werden Sulfonlurn-Tetrafluoroborate eingesetzt, bei denen die Alkylgruppen gleich sind.

Die eingesetzten Sulfonium-Tetrafluoroborate sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Beispiele für Sulfonium-Tetrafluoroborate sind Trimethylsulfonium-, Triethylsulfonium- , Dimethylethylsulfonium-, Diethylmethylsulfonium-, Dipropylmethylsulfonium-, Dipropylethylsulfonium-, Dibutylmethylsulfonium-, Di-*sec*-butylmethylsulfonium-, Dibutylethylsulfonium-Tetrafluoroborat. Ganz besonders bevorzugt wird Trimethylsulfonium- und Triethylsulfonium-Tetrafluoroborat eingesetzt.

Das erfindungsgemäße Verfahren kann in einem Lösungsmittel oder ohne Zusatz eines Lösungsmittels durchgeführt werden. Ein geeignetes Lösungsmittel ist beispielsweise Dichlormethan oder Diethylether.

Vorzugsweise erfolgen die Verfahren ohne Zusatz eines Lösungsmittels.

Die Reaktionstemperaturen liegen im Bereich von 0°C bis 150°C, vorzugsweise im Bereich von 50 bis 100°C.

Anionen, wobei x, y, z, n, R^{f1}, R^{f2} und w die vorab angegebene Bedeutung haben. Viele der unter Verwendung der erfindungsgemäßen Oxoniumsalze und Sulfoniumsalze hergestellten Oniumsalze sind ionische Flüssigkeiten und damit für viele Anwendungen von großem Interesse. Insgesamt wird durch die Bereitstellung der erfindungsgemäßen Oxoniumsalze und Sulfoniumsalze ein alternativer Zugang zu gängigen ionischen Flüssigkeiten bereitgestellt, beziehungsweise die Synthese einzelner ionischer Flüssigkeiten überhaupt ermöglicht. Dies betrifft insbesondere diejenigen ionischen Flüssigkeiten, bei denen das Kation in den bei den üblichen Synthesemethoden verwendeten wässrigen Medien nicht stabil ist.

Das organische Kation der Oniumsalze kann ein Tritylium-, Ammonium-, Phosphonium-, Guanidinium, Uronium-, Thiouronium-Kation oder ein heterocyclisches Kation sein.

Im einfachsten Fall erfolgt die Herstellung der entsprechenden OniumSalze mit [PFₓ(C_{y}F_{2y+1-z}H_{z})₆₋ₓ]⁻-, [BFₙ(CN)₄₋ₙ]⁻-, [(R^{f1}SO₂)₂N]⁻- oder [BF_{w}R^{f2}_{4-w}]⁻-Anionen durch Umsetzung eines entsprechenden Onium-Halogenids des organischen Kations mit einem entsprechenden Oxoniumsalz oder Sulfoniumsalz gemäß der vorliegenden Erfindung. Darüber hinaus eignen sich auch die entsprechenden Onium-Tetrafluoroborate oder - Hexafluorophosphate für die Umsetzung mit einem erfindungsgemäßen Oxonium- oder Sulfoniumsalz.

Ammonium- oder Phosphoniumhalogenide können beispielsweise durch die Formel (1)

[XR₄]⁺ Hal⁻ (1),

beschrieben werden,
wobei
X N, P
Hal Cl, Br oder I und
R jeweils unabhängig voneinander
H, wobei nicht alle Substituenten R gleichzeitig H sein dürfen, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann bedeutet,
wobei ein oder mehrere R teilweise oder vollständig mit F substituiert sein können, wobei jedoch nicht alle vier oder drei R vollständig mit F substituiert sein dürfen,
und wobei ein oder zwei nicht benachbarte und nicht α-ständige Kohlenstoffatome des R, durch Atome und/oder Atomgruppierungen ausgewählt aus der Gruppe -O-, -S-, -S(O)-, -SO₂- und -NR'- oder durch die Endgruppe CN, -C(O)X' oder -SO₂X' mit R' = H, nicht, teilweise oder perfluoriertes C₁- bis C₆-Alkyl, C₃- bis C₇-Cycloalkyl, unsubstituiertes oder substituiertes Phenyl, oder unsubstituierter oder substituierter Heterocyclus und X' = OH, F, Cl oder Br, ersetzt sein können.

Ausgeschlossen sind jedoch Verbindungen der Formeln (1), in denen alle vier oder drei Substituenten R vollständig mit Halogenen substituiert sind, beispielsweise Tris(trifluormethyl)methylammoniumchlorid, Tetra(trifluormethyl)ammoniumchlorid oder Tetra(nonafluorbutyl)-ammoniumchlorid,Tris(trifluormethyl)methylphosphoniumchlorid, Tetra(trifluormethyl)phosphoniumchlorid oder Tetra(nonafluorbutyl)-phosphoniumchlorid.

Die entsprechenden Ammonium- oder Phosphoniumtetrafluoroborate oder -Hexafluorophosphate können ebenfalls in analoger Weise eingesetzt werden.

Uronium- oder Thiouroniumhalogenide können beispielsweise durch die Formel (2)

[(R¹R²N)-C(=XR⁷)(NR³R⁴)]⁺ Hal⁻ (2)

und Guanidiniumhalogenide können beispielsweise durch die Formel (3)

[C(NR¹R²)(NR³R⁴)(NR⁵R⁶)]⁺ Hal⁻ (3),

beschrieben werden,
wobei
X bei Formel (2) O oder S bedeutet,
Hal bei Formel (2) Br oder I bedeutet und bei Formel (3) Cl, Br oder I bedeutet, und
R¹ bis R⁷ jeweils unabhängig voneinander
Wasserstoff oder CN, wobei Wasserstoff für R⁷ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁷ teilweise oder vollständig mit F substituiert sein können, wobei jedoch nicht alle Substituenten an einem N-Atom vollständig mit F substituiert sein dürfen, wobei die Substituenten R¹ bis R⁷ paarweise durch Einfach- oder Doppelbindung miteinander verbunden sein können
und wobei ein oder zwei nicht benachbarte und nicht direkt am Heteroatom gebundene Kohlenstoffatome der Substituenten R¹ bis R⁶ durch Atome
und/oder Atomgruppierungen ausgewählt aus der Gruppe -O-, -S-, -S(O)-, - SO₂- und -NR'- oder durch die Endgruppe CN, -C(O)X' oder -SO₂X' mit R' = H, nicht, teilweise oder perfluoriertes C₁- bis C₆-Alkyl, C₃- bis C₇-Cycloalkyl, unsubstituiertes oder substituiertes Phenyl, oder unsubstituierter oder substituierter Heterocyclus und X' = OH, F, Cl oder Br, ersetzt sein können.

Halogenide mit heterocyclischem Kation können beispielsweise durch die Formel (4)

[HetN]⁺ Hal⁻ (4)

beschrieben werden, wobei
Hal Cl, Br oder I und
HetN⁺ ein heterocyclisches Kation, ausgewählt aus der Gruppe 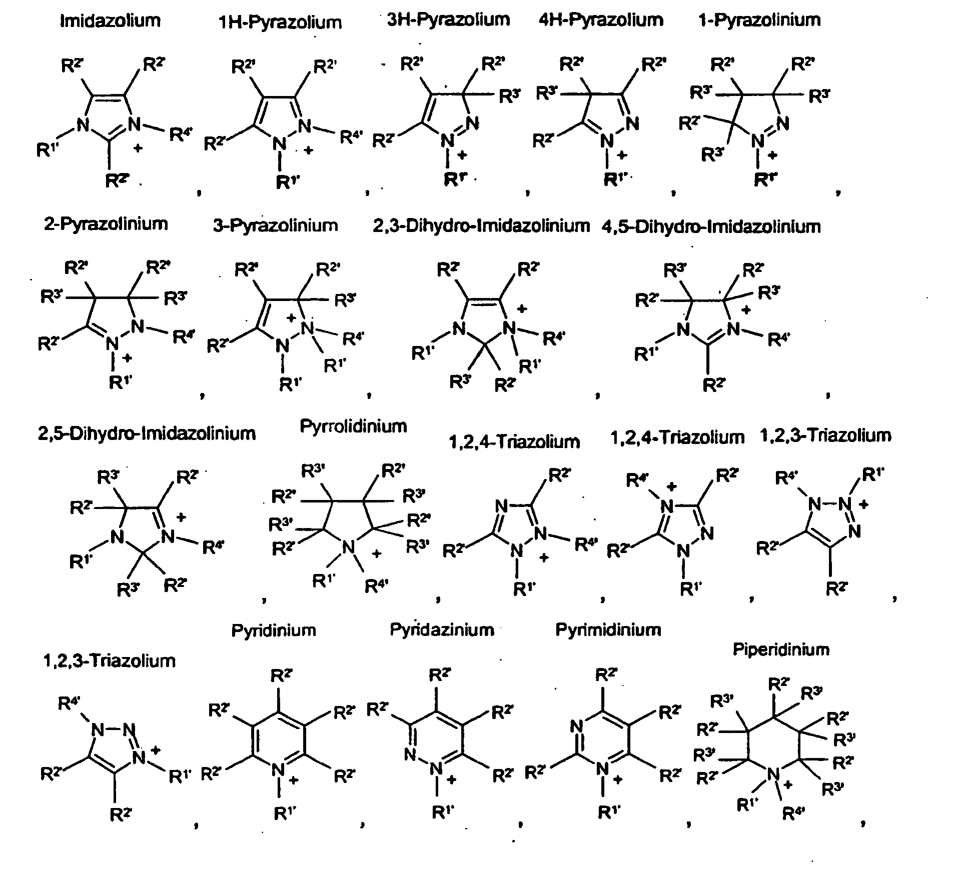 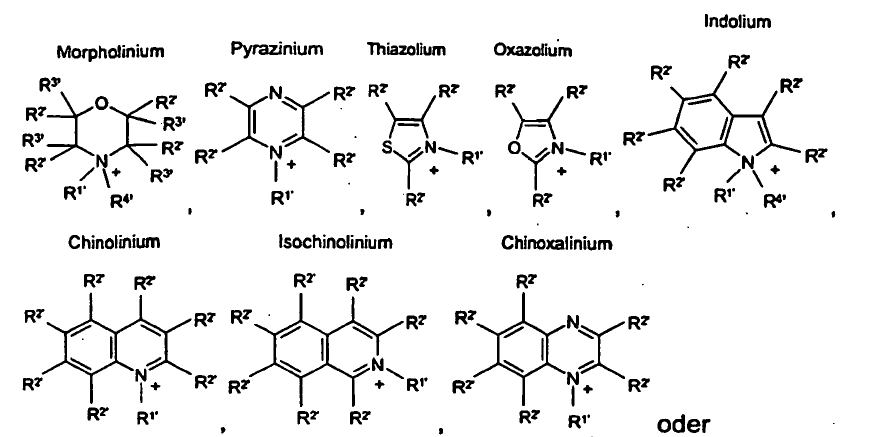 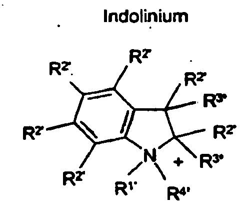 bedeutet, wobei die Substituenten
R¹' bis R⁴' jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt.
gesättigtes, teilweise oder vollständig ungesättigtes Cycloalkyl mit 3-7 C-Atomen, das mit Alkylgruppen mit 1-6 C-Atomen substituiert sein kann oder Aryl-C₁-C₆-alkyl bedeutet,
wobei die Substituenten R¹' und R⁴' teilweise oder vollständig mit F substituiert sein können, wobei jedoch nicht gleichzeitig R¹' und R⁴' vollständig mit F substituiert sein dürfen,
wobei die Substituenten R¹' bis R⁴' teilweise oder vollständig mit OR',
N(R')₂, CN oder Halogen substituiert sein können und wobei ein oder zwei nicht benachbarte und nicht zum Heteroatom α-ständige Kohlenstoffatome von R¹' bis R⁴' durch Atome und/oder Atomgruppierungen ausgewählt aus der Gruppe -O-, -S-, -SO₂- und -NR'- oder durch die Endgruppe CN, - C(O)X' oder -SO₂X' mit R' = H, nicht, teilweise oder perfluoriertes C₁- bis C₆-Alkyl, C₃- bis C₇-Cycloalkyl, unsubstituiertes oder substituiertes Phenyl, oder unsubstituierter oder substituierter Heterocyclus und X' = OH, F, Cl oder Br, ersetzt sein können.

Unter vollständig ungesättigten Substituenten werden im Sinne der vorliegenden Erfindung auch aromatische Substituenten verstanden.

Als Substituenten R und R¹ bis R⁷ der Verbindungen der Formeln (1) bis (3) kommen erfindungsgemäß dabei neben Wasserstoff bevorzugt in Frage: C₁- bis C₂₀-, insbesondere C₁- bis C₁₄-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl. Die Substituenten R und R¹ bis R⁷ können jedoch ebenfalls durch weitere funktionelle Gruppen substituiert sein, beispielsweise durch CN, SO₂R', SO₂OR' oder COOR'. R' bedeutet nicht, teilweise oder perfluoriertes C₁- bis C₆ Alkyl, C₃- bis C₇-Cycloalkyl, unsubstituiertes oder substituiertes Phenyl.

Die Substituenten R in den Verbindungen der Formel (1) können dabei gleich oder verschieden sein. Bevorzugt sind drei Substituenten in Formel (1) gleich und ein Substituent verschieden.

Der Substituent R ist insbesondere bevorzugt Methyl, Ethyl, Isopropyl, Propyl, Butyl, sek.-Butyl, Pentyl, Hexyl, Octyl, Decyl, ,Tetradecyl oder Alkoxyalkyl, worin Alkoxy ein -O-(C₁-C₈-Alkyl) bedeutet,.

Bis zu vier Substituenten des Guanidinium-Kations können auch paarweise derart verbunden sein, dass mono-, bi- oder polycyclische Kationen entstehen.

Ohne Einschränkung der Allgemeinheit sind Beispiele für solche Guanidinium-Kationen: 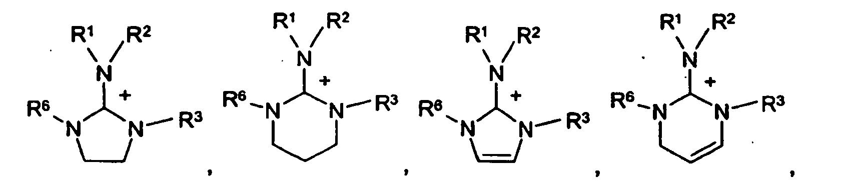 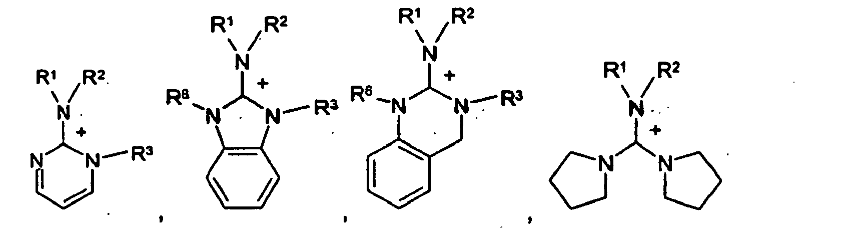 oder 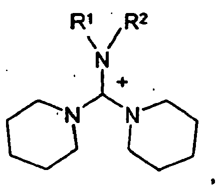 wobei die Substituenten R¹ bis R³ und R⁶ eine zuvor angegebene oder besonders bevorzugte Bedeutung haben können.

Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, C₁-C₆-Alkoxy, SCF₃, SO₂CH₃, SO₂CF₃, COOR", SO₂NR"₂, SO₂X', SO₃R", substituiertes oder unsubstituiertes Phenyl substituiert sein, wobei X' und R" eine zuvor oder später angegebene Bedeutung haben.

Bis zu vier Substituenten des des Uroniums- oder Thiouroniumkations [(R¹R²N)-C(=XR⁷)(NR³R⁴)]⁺ können auch paarweise derart verbunden sein, dass mono-, bi- oder polycyclische Kationen entstehen.

Ohne Einschränkung der Allgemeinheit sind Beispiele für solche Kationen im Folgenden angegeben: 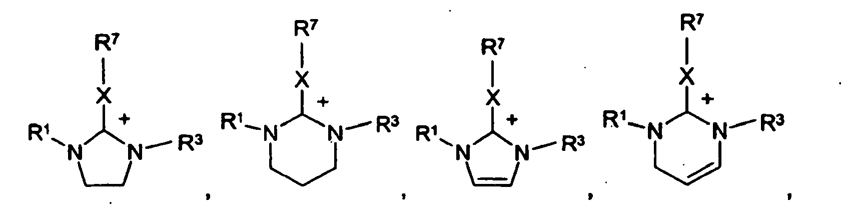 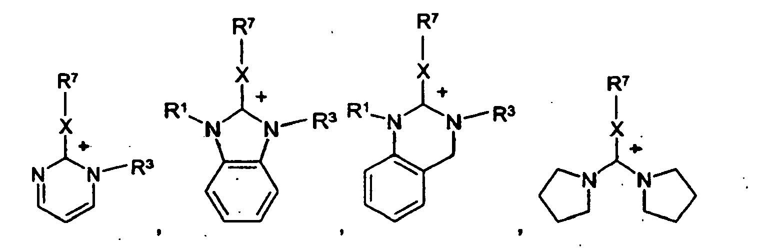 oder 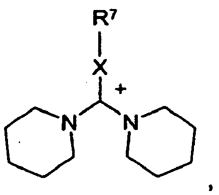 wobei X = O oder S bedeutet und die Substituenten, R¹, R³ und R⁷ eine zuvor angegebene oder besonders bevorzugte Bedeutung haben können.

Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, C₁-C₆-Alkoxy, SCF₃, SO₂CH₃, SO₂CF₃, COOR", SO₂NR"₂, SO₂X', SO₃R", substituiertes oder unsubstituiertes Phenyl substituiert sein, wobei X' und R" eine zuvor oder später angegebene Bedeutung haben.

Die C₁-C₁₄-Alkylgruppe ist beispielsweise Methyl, Ethyl, Isopropyl, Propyl, Butyl, sek.-Butyl oder tert.-Butyl, femer auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl oder Tetradecyl. Gegebenenfalls perfluoriert, beispielsweise als Difluormethyl, Trifluormethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Vinyl, Allyl, 2-, oder 3-Butenyl, Isobutenyl, sek.-Butenyl, femer 4-Pentenyl, iso-Pentenyl, Hexenyl, Heptenyl, Octenyl, -C₉H₁₇, -C₁₀H₁₉ bis -C₂₀H₃₉; vorzugsweise Allyl, 2- oder 3-Butenyl, Isobutenyl, sek.-Butenyl, femer bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, femer 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Aryl-C₁-C₆-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit F substituiert sein können, besonders bevorzugt Benzyl oder Phenylpropyl.

Der Phenylring oder auch die Alkylenkette können jedoch ebenfalls durch weitere funktionelle Gruppen substituiert sein, beispielsweise durch CN, SO₂R', SO₂OR' oder COOR'. R' hat dabei eine zuvor definierte Bedeutung.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit C₁- bis C₆-Alkylgruppen substituiert sein können, wobei wiederum die Cycloalkylgruppe oder die mit C₁- bis C₆-Alkylgruppen substituierte Cycloalkylgruppe auch mit Halogenatomen wie F, Cl, Br oder I, insbesondere F oder Cl oder NO₂ substituiert sein kann. Die Cycloalkylgruppen können jedoch ebenfalls durch weitere funktionelle Gruppen substituiert sein, beispielsweise durch CN, SO₂R', SO₂OR' oder COOR'. R' hat dabei eine zuvor definierte Bedeutung.

In den Substituenten R, R¹ bis R⁶ oder R^{1'} bis R^{4'} können auch ein oder zwei nicht benachbarte und nicht α-ständig zum Heteroatom gebundene Kohlenstoffatome, durch Atome und/oder Atomgruppierungen ausgewählt aus der Gruppe -O-, -S-, -S(O)-, -SO₂- und -NR'- ersetzt sein.

Ohne Einschränkung der Allgemeinheit sind Beispiele für derart modifizierte Substituenten R, R¹ bis R⁶ und R^{1'} bis R^{4'}:
-OCH₃, -OCH(CH₃)₂, -CH₂OCH₃, -CH₂-CH₂-O-CH₃, -C₂H₄OCH(CH₃)₂, -C₂H₄SC₂H₅, -C₂H₄SCH(CH₃)₂, -S(O)CH₃, -SO₂CH₃, -SO₂C₆H₅, -SO₂C₃H₇, -SO₂CH(CH₃)₂, -SO₂CH₂CF₃, -CH₂SO₂CH₃, -O-C₄H₈-O-C₄H₉, -CF₃, -C₂F₅, -C₃F₇, -C₄F₉, -CF₂CF₂H, -CF₂CHFCF₃, -CF₂CH(CF₃)₂, -C₂F₄N(C₂F₅)C₂F₅, -CHF₂, -CH₂CF₃, -C₂F₂H₃, -C₃FH₆, -CH₂C₃F₇, -CH₂C(O)OCH₃, -CH₂C₆H₅ oder -C(O)C₆H₅.

In R' ist C₃- bis C₇-Cycloalkyl beispielweise Cyclopropyl, Cyclobutyl, Cyclopentyl, Cyclohexyl oder Cycloheptyl.

In R' bedeutet substituiertes Phenyl, durch C₁- bis C₆-Alkyl, C₁- bis C₆-Alkenyl, NO₂, F, Cl, Br, I, C₁-C₆-Alkoxy, SCF₃, SO₂CH₃, SO₂CF₃, COOR", SO₂X', SO₂NR"₂ oder SO₃R" substituiertes Phenyl, wobei X' F, Cl oder Br und R" ein nicht oder teilweise fluoriertes C₁- bis C₆-Alkyl oder C₃- bis C₇-Cycloalkyl wie für R' definiert bedeutet, beispielsweise, o-, m- oder p-Methylphenyl, o-, m- oder p-Ethylphenyl, o-, m- oder p-Propylphenyl, o-, m- oder p-Isopropylphenyl, o-, m- oder p-tert.-Butylphenyl, o-, m- oder p-Nitrophenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-Iodphenyl, weiter bevorzugt 2,3-, 2,4-, 2,5-, 2,6-, 3,4- oder 3,5-Dimethylphenyl, 2,3-, 2,4-, 2,5- , 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

Die Substituenten R¹ bis R⁷ sind jeweils unabhängig voneinander bevorzugt eine geradkettige oder verzweigte Alkylgruppe mit 1 bis 10 C-Atomen. Die Substituenten R¹ und R², R³ und R⁴ und R⁵ und R⁶ in Verbindungen der Formeln (2) und (3) können dabei gleich oder verschieden sein. Besonders bevorzugt sind R¹ bis R⁷ jeweils unabhängig voneinander Methyl, Ethyl, n-Propyl, Isopropyl, n-Butyl, sek.-Butyl, Phenyl oder Cyclohexyl, ganz besonders bevorzugt Methyl, Ethyl, n-Propyl, Isopropyl oder n-Butyl.

Als Substituenten R^{1'} bis R^{4'} von Verbindungen der Formel (4) kommen erfindungsgemäß dabei neben Wasserstoff bevorzugt in Frage: CN, C₁- bis C₂₀-, insbesondere C₁- bis C₁₂-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl oder Aryl-C₁-C₆-alkyl oder Diaminoalkyl mit C₁-C₄-Alkylgruppen, sofern dieses nicht an das Heteroatom gebunden vorliegt.

Die Substituenten R^{1'} bis R^{4'}, insbesondere R^{2'} und R^{3'}, können jedoch ebenfalls durch weitere funktionelle Gruppen substituiert sein, beispielsweise durch CN, SO₂R', SO₂OR' oder COOR'. R' bedeutet H, nicht, teilweise oder perfluoriertes C₁- bis C₆-Alkyl, C₃- bis C₇-Cycloalkyl, unsubstituiertes oder substituiertes Phenyl, oder unsubstituierter oder substituierter Heterocyclus.

Die Substituenten R^{1'} und R^{4'} sind jeweils unabhängig voneinander insbesondere bevorzugt CN, Alkoxyethyl, worin Alkoxy -O-(C₁-C₈-Alkyl) bedeutet, insbesondere Methoxyethyl, Methyl, Ethyl, Isopropyl, Propyl, Butyl, sek.-Butyl, Pentyl, Hexyl, Octyl, Decyl, Cyclohexyl, Phenyl, Phenylpropyl oder Benzyl. Sie sind ganz besonders bevorzugt CN, Methyl, Ethyl, n-Butyl oder Hexyl. In Pyrrolidinium-, Piperidinium oder Indolinium-Verbindungen sind die beiden Substituenten R^{1'} und R^{4'} bevorzugt unterschiedlich.

Der Substituent R^{2'} oder R^{3'} ist jeweils unabhängig voneinander insbesondere Wasserstoff, Methyl, Ethyl, Isopropyl, Propyl, Butyl, sek.-Butyl, tert.-Butyl, Cyclohexyl, Dimethylamino, Diethylamino, Methylethylamino, Phenyl oder Benzyl. Besonders bevorzugt ist R^{2'} Dimethylamino, Wasserstoff, Methyl, Ethyl, Isopropyl, Propyl, Butyl, sek.-Butyl oder tert.-Butyl. Ganz besonders bevorzugt sind R^{2'} und R^{3'} Wasserstoff, Dimethylamino oder Methyl.

HetN⁺ der Formel (4) ist bevorzugt 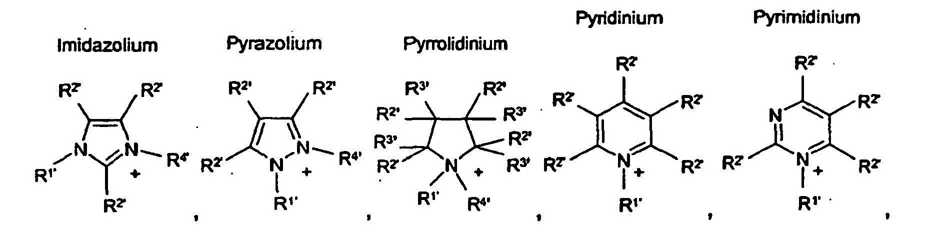 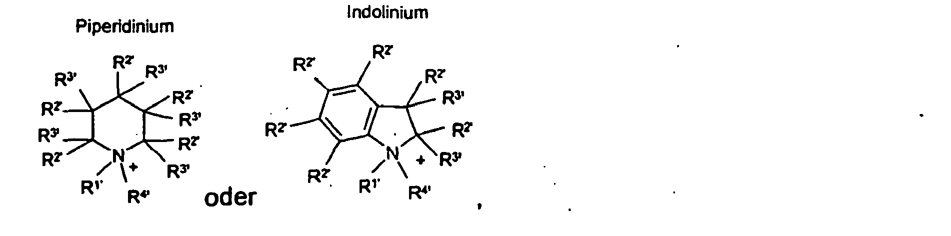 wobei die Substituenten R^{1'} bis R^{4'} jeweils unabhängig voneinander eine zuvor beschriebene Bedeutung haben.

HetN⁺ ist besonders bevorzugt Imidazolium, Pyrrolidinium oder Pyridinium, wie zuvor definiert, wobei die Substituenten R^{1'} bis R^{4'} jeweils unabhängig voneinander eine zuvor beschriebene Bedeutung haben.

Die Reaktion zwischen den Oxoniumsalzen und den Onium-Halogeniden, Onium-Tetrafluoroboraten, oder -Hexafluorophosphaten wird üblicherweise bei Temperaturen zwischen 0°C und 100°C, bevorzugt bei 20°C bis 60°C, besonders bevorzugt bei Raumtemperatur durchgeführt. Bei Umsetzung mit den erfindungsgemäßen Sulfonium-Salzen erfolgt die Reaktion bei Temperaturen zwischen 0°C und 150°C, bevorzugt bei 20°C bis 100°C. Es wird kein Lösungsmittel benötigt. Es können jedoch auch Lösungsmittel eingesetzt werden, beispielsweise Dimethoxyethan, Acetonitril, Dichlormethan, Tetrahydrofuran, Dimethylsulfoxid, Dioxan, Propionitril oder Gemische untereinander.

Die Umsetzung wird mit einem Überschuss oder equimolarer Menge an dem entsprechenden erfindungsgemäßen Oxonium-Salz bzw. SulfoniumSalz durchgeführt.

Die Reaktion kann bei Drücken zwischen Normaldruck und 0.1 mbar erfolgen. Um die Reaktion zu beschleunigen werden vorzugsweise reduzierte Drücke bis 0.1 mbar eingesetzt. Dies gilt insbesondere für Umsetzungen von Onium-Bromiden oder -Iodiden.

Neben der Verwendung zur Herstellung von ionischen Flüssigkeiten eignen sich die erfindungsgemäßen Oxoniumsalze und Sulfoniumsalze auch als Alkylierungsmittel oder als Katalysatoren für kationische Polymerisationen oder photochemisch induzierte Polymerisationen, wobei Sulfoniumsalze mit Triphenylsulfoniumkationen besonders geeignet erscheinen.

Ebenfalls Gegenstand der vorliegenden Erfindung sind ausgewählte Salze, die, wie oben beschrieben, durch Umsetzung mit den erfindungsgemäßen Oxonium- und Sulfoniumsalzen erhalten werden können.

Demgemäß ist ein weiterer Gegenstand der vorliegenden Erfindung die Bereitstellung von Salzen (I) mit Kationen ausgewählt aus der Gruppe umfassend 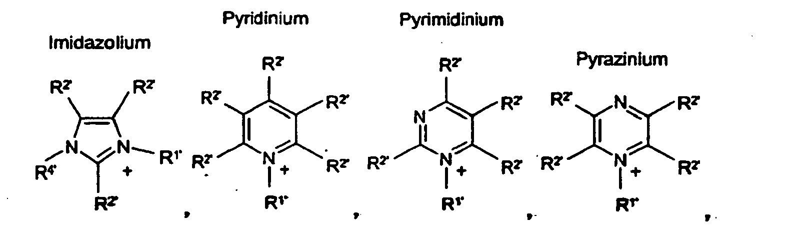 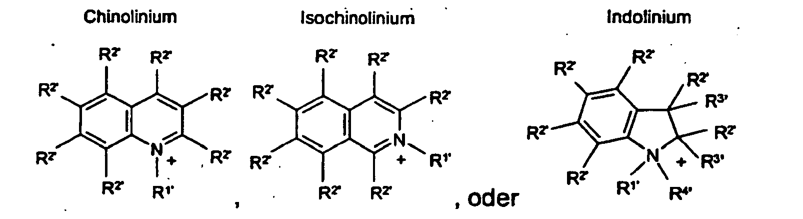 wobei der Substituent
R¹' CN bedeutet und R²' bis R⁴' jeweils unabhängig voneinander die Bedeutung haben
Wasserstoff,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7 C-Atomen, das mit Alkylgruppen mit 1-6 C-Atomen substituiert sein kann oder Aryl-C₁-C₆-alkyl bedeutet,
wobei die Substituenten R²' und R⁴' teilweise oder vollständig mit F substituiert sein können,
wobei die Substituenten R²' und R³' teilweise oder vollständig mit Halogenen oder teilweise mit NO₂ oder CN substituiert sein dürfen,
und wobei ein oder zwei nicht benachbarte und nicht direkt am Heteroatom gebundene Kohlenstoffatome der Substituenten R²' bis R⁴', durch Atome und/oder Atomgruppierungen ausgewählt aus der Gruppe -O-, -S-, -S(O)- oder -SO₂- ersetzt sein können,
und Anionen ausgewählt aus der Gruppe von [PFₓ(C_{y}F_{2y+1-z}H_{z})₆₋ₓ]⁻-Anionen mit 2≤x≤5, 1≤y≤8 und 0≤z≤2y+1,
oder Anionen ausgewählt aus der Gruppe von [BFₙ(CN)₄₋ₙ]⁻-Anionen mit n = 0, 1, 2 oder 3,
oder Anionen ausgewählt aus der Gruppe von [(R^{f1}SO₂)₂N]⁻-Anionen, wobei R^{f1} F oder perfluoriertes und geradkettiges oder verzweigtes Alkyl mit 1-20 C-Atomen, perfluoriertes und geradkettiges oder verzweigtes Alkenyl mit 2-20 C-Atomen und einer oder mehreren Doppelbindungen, perfluoriertes und geradkettiges oder verzweigtes Alkinyl mit 2-20 C-Atomen und einer oder mehreren Dreifachbindungen, perfluoriertes und gesättigtes, teilweise oder vollständig ungesättigtes Cycloalkyl mit 3-7 C-Atomen, das mit Perfluoralkylgruppen substituiert sein kann, bedeutet,
oder Anionen ausgewählt aus der Gruppe von [BF_{w}R^{f2}_{4-w}]⁻-Anionen mit w = 0, 1, 2 oder 3, wobei R^{f2} perforiertes und geradkettiges oder verzweigtes Alkyl mit 1-20 C-Atomen, perfluoriertes und geradkettiges oder verzweigtes Alkenyl mit 2-20 C-Atomen und einer oder mehreren Doppelbindungen, perfluoriertes und geradkettiges oder verzweigtes Alkinyl mit 2-20 C-Atomen und einer oder mehreren Dreifachbindungen, perfluoriertes und gesättigtes, teilweise oder vollständig ungesättigtes Cycloalkyl mit 3-7 C-Atomen, das mit Perfluoralkylgruppen substituiert sein kann, bedeutet, wobei R^{f1} oder R^{f2} jeweils gleich oder verschieden sein können,
wobei R^{f1} oder R^{f2} paarweise durch Einfach- oder Doppelbindungen miteinander verbunden sein können,
und wobei ein oder zwei nicht benachbarte und nicht zum Heteroatom αständige Kohlenstoffatome von R^{f1} oder R^{f2} durch Atome und/oder Atomgruppierungen ausgewählt aus der Gruppe -O-, -SO₂- und -NR'- oder durch die Endgruppe -SO₂X' mit R' = nicht, teilweise oder perfluoriertes C₁- bis C₆-Alkyl, C₃- bis C₇-Cycloalkyl, unsubstituiertes oder substituiertes Phenyl, oder unsubstituierter oder substituierter Heterocyclus und X' = F, Cl oder Br, ersetzt sein können.

Vorzugsweise ist das Anion der Salze (I) ausgewählt aus der Gruppe umfassend [(CF₃SO₂)₂N]⁻, [(C₂F₅SO₂)₂N]⁻, [(FSO₂)₂N]⁻, [P(C₂F₅)₃F₃]⁻, [P(CF₃)₃F₃]⁻, [P(C₂F₄H)(CF₃)₂F₃]⁻, [P(C₂F₃H₂)₃F₃]⁻, [P(C₂F₅)(CF₃)₂F₃]⁻, [P(C₃F₇)₃F₃]⁻, [P(C₄F₉)₃F₃]⁻. [P(C₂F₅)₂F₄]⁻, [BF₃(CF₃)]⁻, [BF₂(C₂F₅)₂]⁻, [BF₃(C₂F₅)]⁻, [BF₂(CF₃)₂]⁻, [B(C₂F₅)₄]⁻, [BF₃(CN)]⁻, [BF₂(CN)₂]⁻, [B(CN)₄]⁻, [B(CF₃)₄]⁻-Anionen.

Bei den genannten Salzen (I) handelt es sich um N-Cyanopyridiniumsalze, N-Cyanoimidazoliumsalze, N-Cyanopyrazinium-salze, N-Cyanopyrimidiniumsalze, N-Cyanochinoliniumsalze, N-Cyanoisochinoliniumsalze, N-Cyanoindoliumsalze, die vielfältig eingesetzt werden können.

In den genannten Salzen (I) ist R²' bis R⁴' vorzugsweise ausgewählt aus Wasserstoff, der nicht am Stickstoff gebunden vorliegt, geradkettigem oder verzweigte Alkyl mit 1-20 C-Atomen oder aus Dialkylaminogruppen, worin Alkyl C₁-C₄-Alkyl bedeutet.

Ganz besonders bevorzugt handelt es sich bei den Salzen (I) um die Verbindungen 1-Cyano-4-dimethylaminopyridiniumtris(pentafluorethyl)trifluorphosphat, 1-Cyano-4-dimethylaminopyridiniumbis(trifluormethylsulfony)imid, 1-Cyano-4-dimethylaminopyridiniumtetracyanoborat und 1-Cyano-4-dimethylaminopyridinium(pentafluoroethyl)trifluorborat.

Weiterhin sind ebenfalls Gegenstand der Erfindung Salze (II) mit Kationen ausgewählt aus der Gruppe umfassend 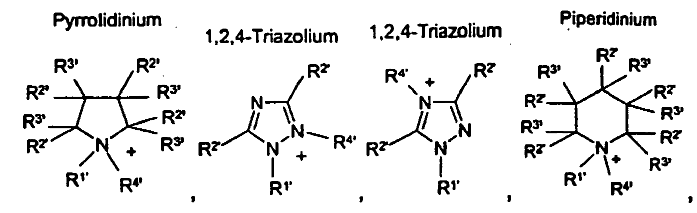 oder 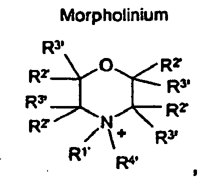 wobei die Substituenten R¹' bis R⁴' jeweils unabhängig voneinander die Bedeutung haben Wasserstoff oder CN, geradkettiges oder verzweigtes Alkyl mit 1-20 C-Atomen, geradkettiges oder verzweigtes Alkenyl mit 2-20 C-Atomen und einer oder mehreren Doppelbindungen, geradkettiges oder verzweigtes Alkinyl mit 2-20 C-Atomen und einer oder mehreren Dreifachbindungen, Dialkylamino mit Alkylgruppen mit 1-4 C-Atomen, welches jedoch nicht am Heteroatom des Heterocyclus gebunden vorliegt, gesättigtes, teilweise oder vollständig ungesättigtes Cycloalkyl mit 3-7 C-Atomen, das mit Alkylgruppen mit 1-6 C-Atomen substituiert sein kann oder Aryl-C₁-C₆-alkyl bedeutet, wobei die Substituenten R¹' und R⁴' teilweise oder vollständig mit F substituiert sein können, wobei jedoch nicht gleichzeitig R¹' und R⁴' CN sind oder vollständig mit F substituiert sein dürfen, wobei die Substituenten R^{2'} und R^{3'} teilweise oder vollständig mit Halogenen oder teilweise mit NO₂ oder CN substituiert sein dürfen, und wobei ein oder zwei nicht benachbarte und nicht direkt am Heteroatom gebundene Kohlenstoffatome der Substituenten R¹' bis R⁴', durch Atome und/oder Atomgruppierungen ausgewählt aus der Gruppe -O-, -S-, -S(O)-, -SO₂- und -NR'- oder durch die Endgruppe CN, -C(O)X' oder -SO₂X' mit R' = H, nicht, teilweise oder perfluoriertes C₁- bis C₆-Alkyl, C₃- bis C₇-Cycloalkyl, unsubstituiertes oder substituiertes Phenyl, oder unsubstituierter oder substituierter Heterocyclus und X' = OH, F, Cl oder Br, ersetzt sein können, und Anionen ausgewählt aus der Gruppe von [PFₓ(C_{y}F_{2y+1-z}H_{z})₆₋ₓ]⁻-Anionen mit 2≤x≤5, 1≤y≤8 und 0≤z≤2y+1, wobei 1-Butyl-1-methylpyrrolidiniumtris(pentafluroethyl)trifluorphosphat ausgenommen ist.

Vorzugsweise ist das Anion der Salze (II) ausgewählt aus der Gruppe umfassend [P(C₂F₅)₃F₃]⁻, [P(CF₃)₃F₃]⁻, [P(C₂F₄H)(CF₃)₂F₃]⁻, [P(C₂F₃H₂)₃F₃]⁻, [P(C₂F₅)(CF₃)₂F₃]⁻, [P(C₃F₇)₃F₃]⁻, [P(C₄F₉)₃F₃]⁻ und [P(C₂F₅)₂F₄]⁻.

In den genannten Salzen (II) ist R¹' bis R⁴' vorzugsweise ausgewählt aus Wasserstoff, geradkettigem oder verzweigten Alkyl mit 1-20 C-Atomen, Dialkylamino mit Alkylgruppen mit 1-4 C-Atomen, welches jedoch nicht am Heteroatom des Heterocyclus gebunden vorliegt, und wobei ein oder zwei nicht benachbarte und nicht direkt am Heteroatom gebundene Kohlenstoffatome der Substituenten R¹' bis R⁴', durch Atome und/oder Atomgruppierungen ausgewählt aus der Gruppe -O-, -S-, -S(O)-, -SO₂- und -NR'- oder durch die Endgruppe CN, -C(O)X' oder -SO₂X' mit R' = H, nicht, teilweise oder perfluoriertes C₁- bis C₆-Alkyl, C₃- bis C₇-Cycloalkyl, unsubstituiertes oder substituiertes Phenyl, oder unsubstituierter oder substituierter Heterocyclus und X' = OH, F, Cl oder Br, ersetzt sein können. Beispiele der genannten Substituenten sind bereits vorab genannt. Insbesondere bevorzugt ist in den genannten Salzen (II) R¹' bis R⁴' ausgewählt aus Wasserstoff, geradkettigem oder verzweigten Alkyl mit 1-20 C-Atomen oder Alkoxyalkyl, worin Alkoxy C₁-C₄-Alkoxy und Alkyl C₁-C₈-Alkyl bedeutet.

Ganz besonders bevorzugt handelt es sich bei den Salzen (II) um jene mit Pyrrolidinium-, Piperidinium- und Morpholinium-Kationen.

Weiterhin sind Gegenstand der vorliegenden Erfindung Salze (III) mit kationen ausgewählt aus der Gruppe umfassend 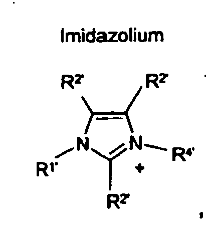 oder 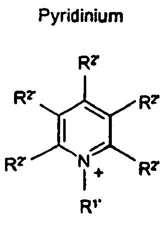 bedeutet,
wobei die Substituenten
R¹' bis R⁴' jeweils unabhängig voneinander die Bedeutung haben geradkettiges oder verzweigtes Alkyl mit 1-20 C-Atomen mit der Maßgabe, dass mindestens einer der Substituenten R¹' oder R⁴' geradkettiges oder verzweigtes Alkyl mit 9-20 C-Atomen ist,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann wobei die Substituenten R¹' und R⁴' teilweise oder vollständig mit F substituiert sein können, wobei jedoch nicht gleichzeitig R¹' und R⁴' vollständig mit F substituiert sein dürfen,
wobei die Substituenten R^{2'} und R^{3'} teilweise oder vollständig mit OR', N(R^{'})₂, CN oder Halogen substituiert sein können und wobei ein oder zwei nicht benachbarte und nicht zum Heteroatom α-ständige Kohlenstoffatome von R¹' bis R⁴' durch Atome und/oder Atomgruppierungen ausgewählt aus der Gruppe -O-, -S-, -SO₂- und -NR'- oder durch die Endgruppe CN, - C(O)X' oder -SO₂X' mit R' = H, nicht, teilweise oder perfluoriertes C₁- bis C₆-Alkyl, C₃- bis C₇-Cycloalkyl, unsubstituiertes oder substituiertes Phenyl, oder unsubstituierter oder substituierter Heterocyclus und X' = OH, F, Cl oder Br, ersetzt sein können und Anionen ausgewählt aus der Gruppe von [PFₓ(C_{y}F_{2y+1-z}H_{z})₆₋ₓ]⁻-Anionen mit 2≤x≤5, 1≤y≤8 und 0≤z≤2y+1, wobei 3-Methyl-1-octadecylimidazoliumtris(pentafluorethyl)trifluorphosphat ausgenommen ist.

Vorzugsweise ist das Anion der Salze (III) ausgewählt aus der Gruppe umfassend [P(C₂F₅)₃F₃]⁻, [P(CF₃)₃F₃]⁻, [P(C₂F₄H)(CF₃)₂F₃]⁻, [P(C₂F₃H₂)₃F₃]⁻, [P(C₂F₅)(CF₃)₂F₃]⁻, [P(C₃F₇)₃F₃]⁻, [P(C₄F₉)₃F₃]⁻ und [P(C₂F₅)F₄]⁻.

In den genannten Salzen (III) ist R¹' bis R⁴' vorzugsweise ausgewählt aus geradkettigem oder verzweigten Alkyl mit 1-20 C-Atomen mit der Maßgabe, dass mindestens einer der Substituenten R¹' oder R⁴' geradkettiges oder verzweigtes Alkyl mit 9-20 C-Atomen ist, wobei die Substituenten R^{2'} und R^{3'} teilweise oder vollständig mit OR', N(R')₂, CN oder Halogen substituiert sein können und wobei ein oder zwei nicht benachbarte und nicht zum Heteroatom α-ständige Kohlenstoflatome von R¹' bis R⁴' durch Atome und/oder Atomgruppierungen ausgewählt aus der Gruppe-O-, -S-, -SO₂- und -NR'- oder durch die Endgruppe CN, -C(O)X' oder -SO₂X' mit R' = H, nicht, teilweise oder perforiertes C₁- bis C₆-Alkyl, C₃- bis C₇-Cycoalkyl, unsubstituiertes oder substituiertes Phenyl, oder unsubstituierter oder substituierter Heterocyclus und X' = OH, F, Cl oder Br, ersetzt sein können. Vorzugsweise ist R¹' bis R⁴' vorzugsweise ausgewählt aus geradkettigem oder verzweigten Alkyl mit 1-20 C-Atomen mit der Maßgabe, dass mindestens einer der Substituenten R¹' oder R⁴' geradkettiges oder verzweigtes Alkyl mit 9-20 C-Atomen ist.

Ganz besonders bevorzugt handelt es sich bei den Salzen (III) um Verbindungen mit Imidazolium-Kationen, bei denen R¹' geradkettiges oder verzweigtes Alkyl mit 1-8 C-Atomen und R⁴' geradkettiges oder verzweigtes Alkyl mit 9-20 C-Atomen bedeutet. Ebenfalls besonders bevorzugt handelt es sich bei den Salzen (III) um Verbindungen mit Pyridinium-Kationen, bei denen R¹' geradkettiges oder verzweigtes Alkyl mit 9-20 C-Atomen und R²' geradkettiges oder verzweigtes Alkyl mit 1-8 C-Atomen bedeutet.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Es versteht sich für den Fachmann von selbst, dass in den vorab und nachfolgend genannten Verbindungen Substituenten wie beispielsweise H, N, O, Cl, F durch die entsprechenden Isotope ersetzt sein können.

Die ¹H-, ¹⁹F- und ³¹P-NMR-Spektren werden an einem Bruker Avance 250 Spektrometer (250.13 MHz für ¹H, 235.36 für ¹⁹F and 101.25 für ³¹P) In Acetonitril-D₃ gemessen, falls nicht anders in den Beispielen angegeben. CCl₃F und TMS werden als interne Referenz bei der Vermessung der ¹⁹F NMR- und Protonen-NMR-Spektren eingesetzt. Für die ³¹P NMR-Spektren wird als externe Referenz 85%-ige H₃PO₄ in D₂O In Acetonitril-D₃ bei einer Frequenz von 230.11 Hz in einem separaten Experiment vermessen.

### Beispiele

### Beispiel 2: Triethylsulfoniumtris(pentafluorethyl)trifluorphosphat

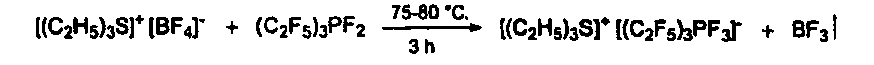

Eine Mischung aus 3.0 g (14.56 mmol) Trlethylsulfoniumtetrafluoroborat und 6.8 g (15.97 mmol) Tris(pentafluorethyl)difluorphosphoran wird auf 75-80°C erhitzt (Temperatur des Ölbades) und unter Stickstoffatmosphäre für drei Stunden gerührt. Flüchtig Bestandteile werden unter reduziertem Druck (7 Pa) bei 75-80°C (Temperatur des Ölbades) innerhalb einer Stunde abgepumpt. Es werden 14.7 g eines Feststoffs erhalten. Die Ausbeute an Triethylsulfoniumtris(pentafluorethyl)trifluorphosphat beträgt 97.4 %, berechnet nach eingesetztem Triethylsulfonlumtetrafluoroborat. Das Produkt wird NMR-spektroskopisch untersucht.
¹H NMR-Spektrum, ppm: 1.40 t (3CH₃), 3.21 q (3CH₂); J³_{H},_{H} = 7.4 Hz.
¹⁹F NMR-Spektrum, ppm: -43.62 dm (PF), -79.67 m (CF₃), -81.36 m (2CF₃). -87.03 dm (PF₂), -115.09 dm (CF₂), -115.62 dm (2CF₂): J¹_{P,F} = 889 Hz, J¹_{P,F} = 906 Hz, J²_{P,F} = 84 Hz, J²_{P,F} = 105 Hz.
³¹P NMR-Spektrum, ppm: -149,0 d,t,m.

### Beispiel 4: Triethylsulfoniumbis(trifluormethylsulfony)imid

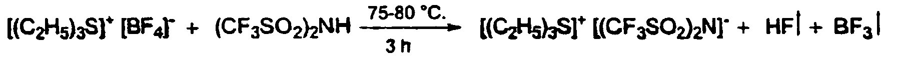

Eine Mischung aus 2.82 g (13.69 mmol) Triethylsulfoniumtetrafluoroborat und 3.85 g (13.69 mmol) Bis(trifluormethylsulfony)imid wird auf 75-80°C erhitzt (Temperatur des Ölbades) und unter Stickstoffatmosphäre für drei Stunden gerührt. Flüchtige Bestandteile werden unter reduziertem Druck (7 Pa) bei 75-80°C (Temperatur des Ölbades) innerhalb einer Stunde abgepumpt. Es werden 5.35 g eines Feststoffs erhalten. Die Ausbeute an Triethylsulfoniumbis(trifluormethylsulfony)imid beträgt 97.9 %. Das Produkt wird NMR-spektroskopisch untersucht.
¹H NMR-Spektrum, ppm: 1.39 t (3CH₃), 3.22 q (3CH₂); J³_{H,H} = 7.4 Hz.
¹⁹F NMR-Spektrum, ppm: -78.89 s (CF₃).

### Beispiel 6: Triethylsulfoniumpentafluorethyltrifluorborat

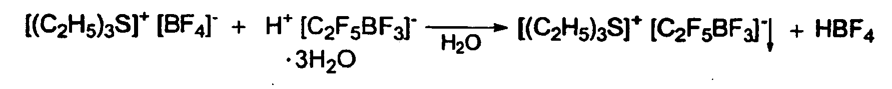

1.3 g (5.34 mmol) Pentafluorethyltrifluorborsäure-Trihydrat, H[C₂F₅BF₃]·3H₂O, wird zu einer Lösung von 1.0 g (4.85 mmol) Triethylsulfoniumtetrafluoroborat in 10 ml Eis-Wasser unter Rühren zugegeben. Die Suspension wird sofort mit Dichlormethan (3 x 20 ml) extrahiert und die erhaltene Lösung mit Magnesiumsulfat getrocknet. Nach Abfiltration von MgSO₄, wird das Dichlormethan im Vakuum (7 Pa) bei Raumtemperatur entfernt und der Rückstand für weitere drei Stunden im Vakuum getrocknet. Es werden 1.24 g eines leicht gelblichen Öls erhalten. Die Ausbeute an Triethylsulfoniumpentafluorethyltrifluorborat beträgt 83.5 %, berechnet nach dem eingesetzten Triethylsulfoniumtetrafluoroborat. Das Produkt wird NMR-spektroskopisch untersucht.
¹H NMR-Spektrum, ppm: 1.39 t (3CH₃), 3.22 q (3CH₂); J³_{H},_{H} = 7.5 Hz.
¹⁹F NMR-Spektrum, ppm: -83.17 q (CF₃), -136.00 q (CF₂), -152.83 q,q (BF₃); J¹_{B,F} = 40.7 Hz, J²_{B,F} = 19.6 Hz, J⁴_{F,F} = 5.0 Hz.
¹¹B NMR-Spektrum, (Referenz: BF₃-Etherat extern) ppm: -0.61 q,t; J¹_{B},_{F} = 40.7 Hz, J²_{B},_{F} = 20.4 Hz.

### Beispiel 7: Triethylsulfoniumpentafluorethyltrifluorborat

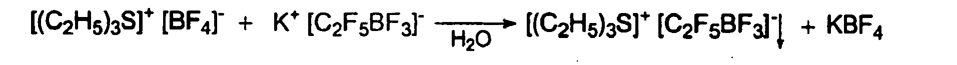

Eine Lösung von 1.03 g (4.56 mmol) Kaliumpentafluorethyltrifluorborat, K[C₂F₅BF₃], in 10 ml Eis-Wasser wird zu einer Lösung von 0.87 g (4.22 mmol) Triethylsulfoniumtetrafluoroborat in 5 ml Eis-Wasser unter Rühren zugegeben. Die Suspension wird sofort mit Dichlormethan (3 x 20 ml) extrahiert und die erhaltene Lösung mit Magnesiumsulfat getrocknet. Nach Abfiltration von MgSO₄, wird das Dichlormethan im Vakuum (7 Pa) bei Raumtemperatur entfernt und der Rückstand für weitere drei Stunden im Vakuum getrocknet. Es werden 1.11 g eines leicht gelblichen Öls erhalten. Die Ausbeute an Triethylsulfoniumpentafluorethyltrifluorborat beträgt 85.9 %, berechnet nach dem eingesetzten Triethylsulfoniumtetrafluoroborat. Das Produkt wird NMR-spektroskopisch untersucht. Die Spektren entsprechen denen gemäß Beispiel 7.

### Beispiel 9: 1-Cyano-4-dimethylaminopyridiniumtris(pentafluorethyl)-trifluorphosphat

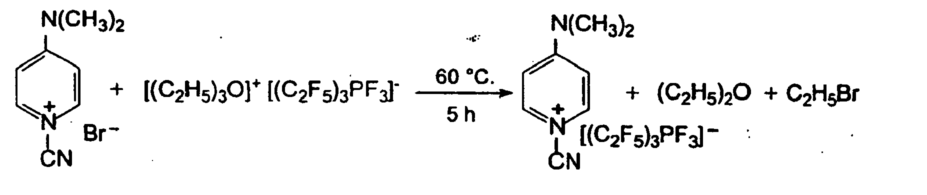

Eine Mischung aus 5.93 g (25.90 mmol) 1-Cyano-4-dimethylaminopyridiniumbromid und 14.30 g (26.09 mmol) Triethyloxonium-tris(pentafluorethyl)trifluorophosphat aus Beispiel 1 wird auf 60°C erhitzt (Temperatur des Ölbades) und unter Stickstoffatmosphäre für fünf Stunden gerührt. Flüchtige Bestandteile werden unter reduziertem Druck (7 Pa) bei 50°C (Temperatur des Ölbades) innerhalb einer Stunde abgepumpt. Es werden 14.32 g eines Feststoffs erhalten. Die Ausbeute an 1-Cyano-4-dimethylaminopyridiniumtris(pentafluorethyl)trifluorphosphat beträgt 93.2 %, bezogen auf das eingesetzte 1-Cyano-4-dimethylaminopyridiniumbromid. Das Produkt wird NMR-spektroskopisch untersucht.
¹H NMR-Spektrum, ppm: 3.34 s (2CH₃), 6.99 d (CH), 7.03 d (CH), 8.04 d (CH), 8.20 d (CH); J³_{H,H} = 8.2 Hz.
¹⁹F NMR-Spektrum, ppm: -43.57 dm (PF), -79.60 m (CF₃), -81.30 m (2CF₃), -87.00 dm (PF₂), -115.05 dm (CF₂), -115.60 dm (2CF₂); J¹_{P,F} = 889 Hz, J¹_{P,F} = 906 Hz, J²_{P,F} = 81 Hz, J²_{P,F} = 107 Hz.

### Beispiel 10: 1-Ethyl-3-methylimidazoliumtris(pentafluorethyl)trifluorphosphat

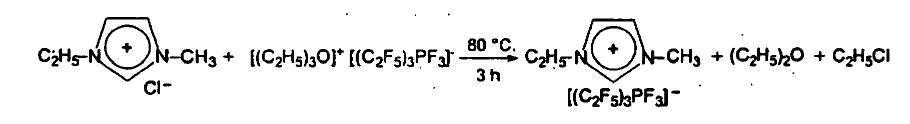

Eine Mischung aus 1.12 g (7.64 mmol) 1-Ethyl-3-methylimidazoliumchlorid und 4.19 g (7.64 mmol) Triethyloxoniumtris(pentafluorethyl)trifluorphosphat aus Beispiel 1 wird auf 70-80 °C erhitzt (Temperatur des Ölbades) und unter Stickstoffatmosphäre für drei Stunden gerührt. Flüchtige Bestandteile werden unter reduziertem Druck (7 Pa) bei 70°C (Temperatur des Ölbades) innerhalb einer Stunde abgepumpt. Es werden 4.20 g einer Flüssigkeit erhalten. Die Ausbeute an 1-Ethyl-3-methylimidazoliumtris(pentafluorethyl)-trifluorphosphat beträgt 98.9 %, bezogen auf das eingesetzte 1-Ethyl-3-methylimidazoliumchlorid. Das Produkt wird NMR-spektroskopisch untersucht.
¹H NMR-Spektrum, ppm: 1.47 t (CH₃), 3.84 s (CH₃), 4.18 q (CH₂), 7.34 m (CH), 7.39 m (CH), 8.43 br. s. (CH); J³_{H,H} = 7.3 Hz.
¹⁹F NMR-Spektrum, ppm: -43.54 dm (PF), -79.60 m (CF₃), -81.29 m (2CF₃), -86.96 dm (PF₂), -115.03 dm (CF₂), -115.55 dm (2CF₂); J¹_{P,F} = 889 Hz, J¹_{P,F} = 906 Hz, J²_{P,F} = 84 Hz, J²_{P,F} = 106 Hz.
³¹P NMR-Spektrum, ppm: -149.0 d,t,m.

### Beispiel 11: 1-Ethyl-3-methylimidazoliumtris(pentafluorethyl)trifluomhosphat

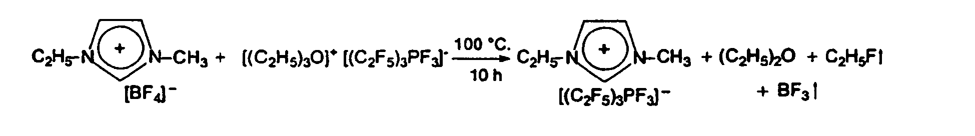

Eine Mischung aus 0.54 g (2.73 mmol) 1-Ethyl-3-methylimidazoliumtetrafluoroborat und 1.50 g (2.74 mmol) Triethyroxoniumtris(pentafluorethyl)-trifluorphosphat aus Beispiel 1 wird auf 100 °C erhitzt (Temperatur des Ölbades) und unter Stickstoffatmosphäre für zehn Stunden gerührt. Flüchtige Bestandteile werden unter reduziertem Druck (7 Pa) bei 100°C (Temperatur des Ölbades) innerhalb einer Stunde abgepumpt. Es werden 1.37 g einer Flüssigkeit erhalten. Die Ausbeute an 1-Ethyl-3-methylimidazoliumtris(pentafluorethyl)trifluorphosphat beträgt 90.3 %, bezogen auf das eingesetzte 1-Ethyl-3-methylimidazoliumtetrafluoroborat. Das Produkt wird NMR-spektroskopisch untersucht.
NMR-Daten: siehe Beispiel 10

### Beispiel 12:1-Ethyl-3-methylimidazoliumtris(aentafluorethyl)trifluorphosphat

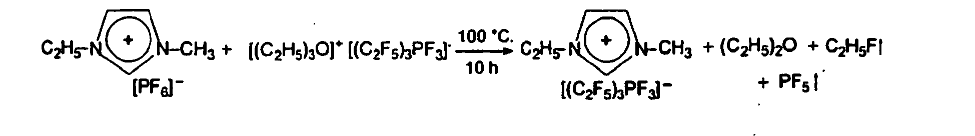

Eine Mischung aus 1.73 g (6.75 mmol) 1-Ethyl-3-methylimidazoliumhexafluorophosphat und 3.70 g (6.75 mmol) Triethyloxoniumtris(pentafluorethyl)-trifluorphosphat aus Beispiel 1 wird auf 100 °C erhitzt (Temperatur des Ölbades) und unter Stickstoffatmosphäre für zehn Stunden gerührt. Flüchtige Bestandteile werden unter reduziertem Druck (7 Pa) bei 100°C (Temperatur des Ölbades) innerhalb einer Stunde abgepumpt. Es werden 3.71 g einer Flüssigkeit erhalten. Die Ausbeute an 1-Ethyl-3-methylimidazoliumtris(pentafluorethyl)trifluorphosphat beträgt 98.7 %, bezogen auf das eingesetzte 1-Ethyl-3-methylimidazoliumhexafluorophosphat. Das Produkt wird NMR-spektroskopisch untersucht.
NMR-Daten: siehe Beispiel 10

### Beispiel 13: Hexamethylguanidiniumtris(pentafluorethyl)trifluorphosphat

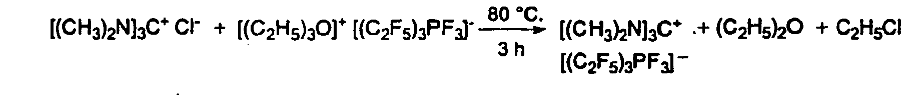

Eine Mischung aus 1.81 g (10.07 mmol) Hexamethylguanidiniumchlorid und 5.53 g (10.09 mmol) Triethyloxoniumtris(pentafluorethyl)trifluorphosphat aus Beispiel 1 wird auf 70-80 °C erhitzt (Temperatur des Ölbades) und unter Stickstoffatmosphäre für drei Stunden gerührt. Flüchtige Bestandteile werden unter reduziertem Druck (7 Pa) bei 70°C (Temperatur des Ölbades) innerhalb einer Stunde abgepumpt. Es werden 5.88 g eines Feststoffs erhalten. Die Ausbeute an Hexamethylguanidiniumtris(pentafluorethyl)trifluorphosphat beträgt 98.9 %, bezogen auf das eingesetzte Hexamethylguanidiniumchlorid. Das Produkt wird NMR-spektroskopisch untersucht.
¹H NMR-Spektrum, ppm: 2.89 s (6CH₃).
¹⁹F NMR-Spektrum, ppm: -43.63 dm (PF), -79.68 m (CF₃), -81.37 m (2CF₃), -87.05 dm (PF₂), -115.07 dm (CF₂), -115.64 dm (2CF₂); J¹_{P,F} = 891 Hz, J¹_{P,F} = 906 Hz, J²_{P,F} = 84 Hz, J²_{P,F} = 105 Hz.
³¹P NMR-Spektrum, ppm: -149.0 d,t,m.

### Beispiel 14: 1-Cyano-4-dimethylaminopyridiniumbis(trifluormethylsulfony)-imid

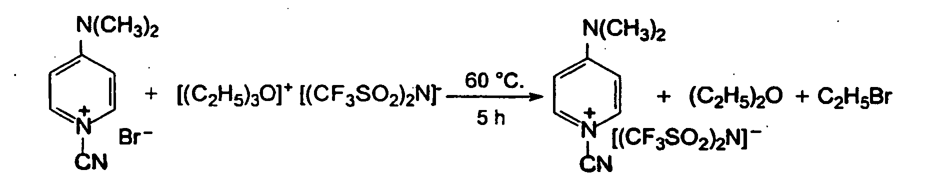

Eine Mischung aus 5.22 g (22.89 mmol) 1-Cyano-4-dimethylaminopyridiniumbromid und 8.77 g (22.88 mmol) Triethyloxonium-bis(trifluormethylsulfony)imid aus Beispiel 3 wird auf 60°C erhitzt (Temperatur des Ölbades) und unter Stickstoffatmosphäre für fünf Stunden gerührt. Flüchtige Bestandteile werden unter reduziertem Druck (7 Pa) bei 50°C (Temperatur des Ölbades) innerhalb einer Stunde abgepumpt. Es werden 14.32 g eines Feststoffs erhalten. Die Ausbeute an 1-Cyano-4-dimethylaminopyridiniumbis(trifluormethylsulfony)imid beträgt 98.2 %, bezogen auf das eingesetzte 1-Cyano-4-dimethylaminopyridiniumbromid. Das Produkt wird NMR-spektroskopisch untersucht.
¹H NMR-Spektrum, ppm: 3.34 s (2CH₃), 6.99 d (CH), 7.03 d (CH), 8.04 d (CH), 8.19 d (CH); J³_{H,H} = 8.2 Hz.
¹⁹F NMR-Spektrum, ppm: -78.96 s.

### Beispiel 15: 1-Ethyl-3-methylimidazoliumbis(trifluormethylsulfony)imid

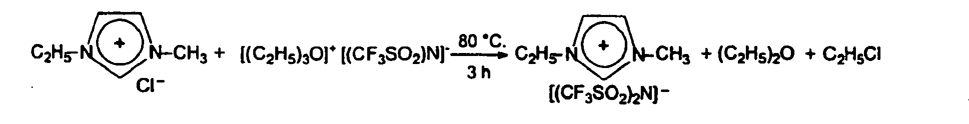

Eine Mischung aus 1.32 g (9.0 mmol) 1-Ethyl-3-methylimidazoliumchlorid und 3.45 g (9.0 mmol) Triethyloxoniumbis(trifluormethylsulfony)imid aus Beispiel 3 wird auf 70-80°C erhitzt (Temperatur des Ölbades) und unter Stickstoffatmosphäre für vier Stunden gerührt. Flüchtige Bestandteile werden unter reduziertem Druck (7 Pa) bei 70°C (Temperatur des Ölbades) innerhalb einer Stunde abgepumpt. Es werden 3.45 g einer Flüssigkeit erhalten. Die Ausbeute an 1-Ethyl-3-methylimidazoliumbis(trifluormethylsulfony)imid beträgt 97.9 %, bezogen auf das eingesetzte 1-Ethyl-3-methylimidazoliumchlorid. Das Produkt wird NMR-spektroskopisch untersucht.
¹H NMR-Spektrum, ppm: 1.45 t (CH₃); 3.83 s (CH₃); 4.17 q (CH₂); 7.37 m (CH); 7.43 m (CH); 8.57 br. s. (CH); ³J_{H,H} = 7.3 Hz.
¹⁹F NMR-Spektrum, ppm: -78.91 s (CF₃).

### Beispiel 16: 1-Ethyl-3-methylimidazoliumtris(pentafluorethyl)trifluorophosphat

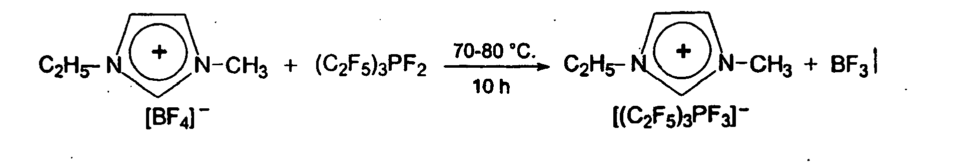

Eine Mischung aus 0.47 g (2.37 mmol) 1-Ethyl-3-methylimidazoliumtetrafluoroborat und 1.12 g (2.63 mmol) Tris(pentafluorethyl)difluorphosphoran wird auf 70-80°C erhitzt (Temperatur des Ölbades) und unter Stickstoffatmosphäre für 10 Stunden gerührt. Flüchtige Bestandteile werden unter reduziertem Druck (7 Pa) bei 70°C (Temperatur des Ölbades) innerhalb einer Stunde abgepumpt. Es werden 1.30 g einer Flüssigkeit erhalten. Die Ausbeute an 1-Ethyl-3-methylimidazoliumtris(pentafluorethyl)-trifluorophosphat beträgt 98.5 %, bezogen auf das eingesetzte 1-Ethyl-3-methylimidazoliumtetrafluoroborat. Das Produkt wird NMR-spektroskopisch untersucht.
NMR-Daten: siehe Beispiel 10

### Beispiel 17: 1-Ethyl-3-methylimidazoliumtris(pentafluoroethyl)trifluorophosphat

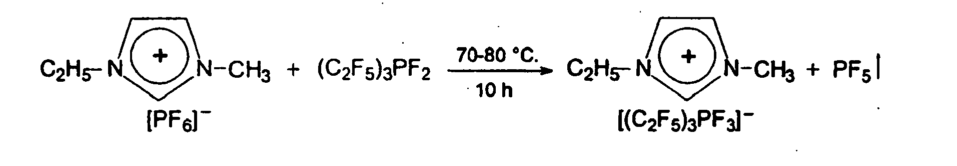

Eine Mischung aus 2.34 g (9.14 mmol) 1-Ethyl-3-methylimidazoliumhexafluorophosphat und 4.28 g (10.05 mmol) Tris(pentafluorethyl)difluorphosphoran wird auf 70-80°C erhitzt (Temperatur des Ölbades) und unter Stickstoffatmosphäre für 10 Stunden gerührt. Flüchtige Bestandteile werden unter reduziertem Druck (7 Pa) bei 70°C (Temperatur des Ölbades) innerhalb einer Stunde abgepumpt. Es werden 4.95 g einer Flüssigkeit erhalten. Die Ausbeute an 1-Ethyl-3-methylimidazoliumtris(pentafluorethyl)-trifluorophosphat beträgt 97.4 %, bezogen auf das eingesetzte 1-Ethyl-3-methylimidazoliumhexafluorophosphat. Das Produkt wird NMR-spektroskopisch untersucht.
NMR-Daten: siehe Beispiel 10

### Beispiel 18: Trityliumtris(pentafluoroethyl)trifluorophosphat

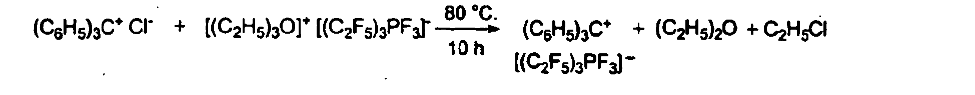

Eine Mischung aus 1.12 g (4.02 mmol) Trityliumchlorid und 2.20 g (4.02 mmol) Triethyloxoniumtris(pentafluorethyl)trifluorphosphat aus Beispiel 1 wird auf 70-80°C erhitzt (Temperatur des Ölbades) und unter Stickstoffatmosphäre für zehn Stunden gerührt. Flüchtige Bestandteile werden unter reduziertem Druck (7 Pa) bei 70°C (Temperatur des Ölbades) innerhalb einer Stunde abgepumpt. Es werden 2.59 g eines Feststoffs erhalten. Die Ausbeute an Trityliumtris(pentafluorethyl)trifluorphosphat beträgt 93.6 %. Das Produkt wird NMR-spektroskopisch untersucht.
¹H NMR-Spektrum, ppm: 7.18-740 m (3C₆H₅);
¹⁹F NMR-Spektrum, ppm: -43.65 dm (PF), -79.69 m (CF₃), -81.39 m (2CF₃), -87.07 dm (PF₂), -115.14 dm (CF₂), -115.68 dm (2CF₂); J¹_{P,F} = 891 Hz, J¹_{P,F} = 902 Hz, J²_{P,F} = 87 Hz, J²_{P,F} = 105 Hz.

### Beispiel 19: 1-Decyl-3-methylimidazoliumtris(pentafluorethyl)-trifluorphosphat

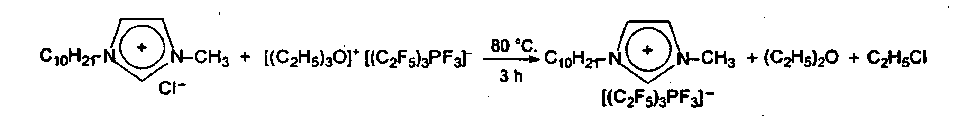

Eine Mischung aus 0.34 g (1.31 mmol) 1-Decyl-3-methylimidazoliumchlorid und 0.72 g (1.31 mmol) Triethyloxoniumtris(pentafluorethyl)trifluorphosphat aus Beispiel 1 wird auf 80°C erhitzt (Temperatur des Ölbades) und unter Stickstoffatmosphäre für drei Stunden gerührt. Flüchtige Bestandteile werden unter reduziertem Druck (7 Pa) bei 80°C (Temperatur des Ölbades) innerhalb einer Stunde abgepumpt. Es werden 0.84 g einer Flüssigkeit erhalten. Die Ausbeute an 1-Decyl-3-methylimidazoliumtris(pentafluorethyl)trifluorphosphat beträgt 96 %. Das Produkt wird NMR-spektroskopisch untersucht.
¹H NMR-Spektrum, ppm: 0.90 m (CH₃), 1.30 m (7CH₂), 1.82 m (CH₂), 3.82 s (CH₃), 4.11 t (CH₂), 7.32 d,d (CH), 7.36 d,d (CH), 8.37 m (CH); J³_{H,H} = 7.4 Hz, J⁴_{H,H} = 1.8 Hz..
¹⁹F NMR-Spektrum, ppm: -43.61 dm (PF), -79.65 m (CF₃), -81.34 m (2CF₃), -87.07 dm (PF₂), -115.09 dm (CF₂), -115.61 dm (2CF₂); J¹_{P,F} = 891 Hz, J¹_{P,F} = 906 Hz, J²_{P,F} = 83 Hz, J²_{P,F} = 105 Hz.
³¹P NMR-Spektrum, ppm: -148.1 d,t,m.

### Beispiel 20: 1-Hexyl-1-methylpyrrolidiniumtris(pentafluorethyl)-trifluorphosphat

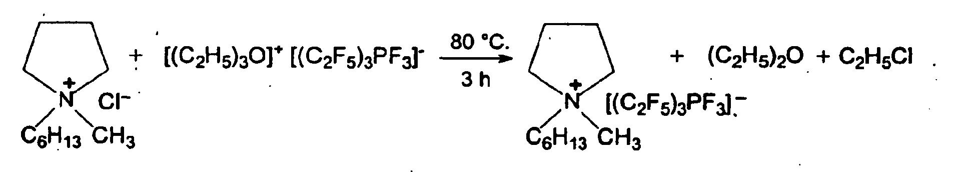

Eine Mischung aus 0.20 g (0.97 mmol) 1-Hexyl-1-methylpyrrolidiniumchlorid und 0.53 g (0.97 mmol) Triethyloxoniumtris(pentafluorethyl)-trifluorphosphat aus Beispiel 1 wird auf 80°C erhitzt (Temperatur des Ölbades) und unter Stickstoffatmosphäre für drei Stunden gerührt. Flüchtige Bestandteile werden unter reduziertem Druck (7 Pa) bei 80°C (Temperatur des Ölbades) innerhalb einer Stunde abgepumpt. Es werden 0.55 g einer Flüssigkeit erhalten. Die Ausbeute an 1-Hexyl-1-methylpyrrolidiniumtris(pentafluorethyl)trifluorphosphat beträgt 93 %. Das Produkt wird NMR-spektroskopisch untersucht.
¹H NMR-Spektrum, ppm: 0.92 m (CH₃), 1.35 m (3CH₂), 1.74 m (CH₂), 2.16 m (2CH₂), 2.93 s (CH₃), 3.18 m (CH₂), 3.40 m (2CH₂).
¹⁹F NMR-Spektrum, ppm: -43.58 dm (PF), -79.64 m (CF₃), -81.34 m (2CF₃), -87.00 dm (PF₂), -115.03 dm (CF₂), -115.58 dm (2CF₂); J¹_{P,F} = 891 Hz, J¹_{P,F} = 906 Hz, J²_{P,F} = 84 Hz, J²_{P,F} = 106 Hz.
³¹P NMR-Spektrum, ppm: -148.1 d,t,m.

Alternative, im Rahmen der vorliegenden Erfindung genannte Verbindungen lassen sich in für den Fachmann nahe liegender Weise analog herstellen.

## Patentansprüche

1. Verfahren zur Herstellung von Sulfoniumsalzen mit [(R°)₃S]⁺-Kationen und mit [PFₓ(CyF_{2y+1-z}Hz)₆₋ₓ]⁻-Anionen, wobei R° geradkettige oder verzweigte Alkylgruppen mit 1-8 C-Atomen oder unsubstituiertes Phenyl oder mit R°, OR°, N(R°)₂, CN oder Halogen substituiertes Phenyl bedeutet, und 2≤x≤5, 1≤y≤8 und 0≤z≤2y+1 bedeutet,
**dadurch gekennzeichnet, dass** ein [(R°)₃S]⁺-Tetrafluoroborat oder - Hexafluorophosphat mit einem Phosphoran der allgemeinen Formel PFₐ(C_{y}F_{2y+1-z}H_{z})₅₋ₐ, wobei 1≤a≤4 umgesetzt wird, wobei als Sulfonium-Tetrafluoroborat mit der Formel [(R⁰)₃S]⁺ [BF₄]⁻ ein Sulfonium-Tetrafluoroborat mit geradkettigen oder verzweigten Alkylgruppen mit 1-8 C-Atomen, die jeweils unabhängig voneinander sind, eingesetzt wird.

2. Verfahren gemäß Anspruch 1, **dadurch gekennzeichnet, dass** die Umsetzung in einem Lösungsmittel oder ohne Zusatz eines Lösungsmittels durchgeführt wird.

3. Verfahren gemäß Anspruch 1 oder 2, **dadurch gekennzeichnet, dass** die Umsetzung bei Temperaturen von 0°C bis 150°C erfolgt.

4. Verfahren gemäß Anspruch 1, **dadurch gekennzeichnet, dass** das [(R°)₃S]⁺-Tetrafluoroborat ausgewählt wird aus der Gruppe Trimethylsulfonium- , Triethylsulfonium- , Dimethylethylsulfonium-, Diethylmethylsulfonium-, Dipropylmethylsulfonium-, Dipropylethylsulfonium-, Dibutylmethylsulfonium-, Di-sec-butylmethylsulfonium-, Dibutylethylsulfonium-Tetrafluoroborat.

## Claims

1. Process for the preparation of sulfonium salts having [(R°)₃S]⁺ cations and having [PFₓ(C_{y}F_{2y+1-z}H_{z})₆₋ₓ]⁻ anions, where R° denotes straight-chain or branched alkyl groups having 1-8 C atoms or unsubstituted phenyl or phenyl which is substituted by R°, OR°, N(R°)₂, CN or halogen, and 2≤x≤5, 1≤y≤8 and 0≤z≤2y+1,
**characterised in that** an [(R°)₃S]⁺ tetrafluoroborate or hexafluorophosphate is reacted with a phosphorane of the general formula PFₐ(C_{y}F_{2y+1-z}H_{z})₅₋ₐ, where 1≤a≤4, where the sulfonium tetrafluoroborate of the formula [(R°)₃S]⁺ [BF₄]⁻ employed is a sulfonium tetrafluoroborate containing straight-chain or branched alkyl groups having 1-8 C atoms, which are in each case independent of one another.

2. Process according to Claim 1, **characterised in that** the reaction is carried out in a solvent or without addition of a solvent.

3. Process according to Claim 1 or 2, **characterised in that** the reaction is carried out at temperatures of 0°C to 150°C.

4. Process according to Claim 1, **characterised in that** the [(R°)₃S]⁺ tetrafluoroborate is selected from the group trimethylsulfonium, triethylsulfonium, dimethylethylsulfonium, diethylmethylsulfonium, dipropylmethylsulfonium, dipropylethylsulfonium, dibutylmethylsulfonium, di-sec-butylmethylsulfonium, dibutylethylsulfonium tetrafluoroborate.

## Revendications

1. Procédé de préparation de sels de sulfonium ayant des cations [(R°)₃S]⁺ et ayant des anions [PFₓ(C_{y}F_{2y+1-z}H_{z})₆₋ₓ]⁻, où R° désigne des groupements alkyle à chaîne linéaire ou ramifiée ayant 1-8 atomes de C ou phényle non substitué ou phényle qui est substitué par R°, OR°, N(R°)₂, CN ou halogène, et 2≤x≤5, 1≤y≤8 et 0≤z≤2y+1,
**caractérisé en ce qu'**un tétrafluoroborate ou hexafluorophosphate de [(R°)₃S]⁺ est réagi avec un phosphorane de formule générale PFₐ(C_{y}F_{2y-1-z}H_{z})₅₋ₐ, où 1≤a≤4, où le tétrafluoroborate de sulfonium de formule [(R°)₃S]⁺ [BF₄]⁻ employé est un tétrafluoroborate de sulfonium contenant des groupements alkyle à chaîne linéaire ou ramifiée ayant 1-8 atomes de C, qui sont dans chaque cas indépendants les uns des autres.

2. Procédé selon la revendication 1, **caractérisé en ce que** la réaction est effectuée dans un solvant ou sans addition de solvant.

3. Procédé selon la revendication 1 ou 2, **caractérisé en ce que** la réaction est effectuée à des températures allant de 0°C à 150°C.

4. Procédé selon la revendication 1, **caractérisé en ce que** le tétrafluoroborate de [(R°)₃S]⁺ est choisi dans le groupe constitué par le tétrafluoroborate de triméthylsulfonium, triéthylsulfonium, diméthyléthylsulfonium, diéthylméthylsulfonium, dipropylméthylsulfonium, dipropyléthylsulfonium, dibutylméthylsulfonium, di-sec-butylméthylsulfonium, dibutyléthylsulfonium.
